# EUROPEAN PATENT APPLICATION

(11) **EP 4 582 517 A1**
(43) Date of publication of application: **09.07.2025**
(21) Application number: 23860399.7
(22) Date of filing: 30.08.2023
(51) Int. Cl.: C10G 2/00, B01D 53/04, B01D 53/22, C01B 32/50, C07C 29/152, C07C 31/04

(54) **LIQUID FUEL PRODUCTION SYSTEM AND LIQUID FUEL PRODUCTION METHOD**

(30) Priority: 02.09.2022 JP 2022140351
(71) Applicant: NGK Insulators, Ltd., Nagoya-shi, Aichi 467-8530 (JP)
(72) Inventor: MAEHARA, Sota, Nagoya-shi, Aichi 467-8530 (JP); IIDA, Kazuki, Nagoya-shi, Aichi 467-8530 (JP); ANDO, Junichi, Nagoya-shi, Aichi 467-8530 (JP); OKUMA, Yusuke, Nagoya-shi, Aichi 467-8530 (JP); KAN, Hirofumi, Nagoya-shi, Aichi 467-8530 (JP); SHIBAGAKI, Yukinari, Nagoya-shi, Aichi 467-8530 (JP); TAKAHASHI, Michio, Nagoya-shi, Aichi 467-8530 (JP)
(74) Representative: Müller-Boré & Partner Patentanwälte PartG mbB
(86) International application number: PCT/JP2023/031433
(87) International publication number: WO 2024/048637

(57) **Abstract**

A primary object of the present invention is to alleviate constraint on a place where a carbon dioxide-capturing system is installed, and/or to stably secure a CO₂ supply source and/or cooling energy in a liquid fuel production system. According to an embodiment of the present invention, provided is a liquid fuel production system including: a gas-adsorbing/desorbing portion configured to adsorb a predetermined gas A, and to desorb the gas A by being heated; a heating medium-supplying portion configured to supply, to the gas-adsorbing/desorbing portion, a heating medium for heating the gas-adsorbing/desorbing portion; a liquid fuel-synthesizing portion including a first gas flow path storing a catalyst that advances a conversion reaction from a raw material gas containing at least carbon dioxide and hydrogen to a liquid fuel, and a second gas flow path through which a temperature-controlling gas that controls a temperature of the first gas flow path is flowed, the liquid fuel-synthesizing portion being configured to flow a first outflow gas out of the first gas flow path, and to flow a second outflow gas out of the second gas flow path; and a heat exchanger configured to perform heat exchange between the first outflow gas and the heating medium to heat the heating medium. The first outflow gas contains a condensable gas.

## Description

### Technical Field

The present invention relates to a liquid fuel production system and a method of producing a liquid fuel.

### Background Art

In recent years, to reduce an environmental load, an attempt has been made to separate and capture carbon dioxide (CO₂) in air or various emission sources. In, for example, Patent Literature 1, there is a disclosure of a carbon dioxide-capturing system that separates and captures CO₂ as follows: the system causes a carbon dioxide adsorbent to adsorb CO₂, and causes the adsorbent to desorb CO₂ through its heating.

In addition, the following has been proposed for the purpose of achieving a carbon neutral society: CO₂ is grasped as a carbon resource, and is converted into a liquid fuel useful as an industrial basic raw material. In, for example, Patent Literature 2, there is a disclosure of a liquid fuel synthesis system that performs a conversion reaction from a raw material gas containing CO₂ and hydrogen (H₂) to methanol with a membrane reactor including a catalyst and a water vapor separation membrane.

### Citation List

### Patent Literature

[PTL 1] WO 2013/119929 A1
[PTL 2] JP 2018-8940 A

### Summary of Invention

### Technical Problem

The carbon dioxide-capturing system that captures CO₂ through its adsorption and desorption requires a heat source at the time of the desorption of CO₂, and hence involves, for example, the following constraint: a place where a heat source such as geothermal energy is present is selected as a place where the system is installed. Accordingly, an area in which the system can be operated is limited.

Meanwhile, in the carbon neutral liquid fuel synthesis system, in addition to hydrogen obtained by water electrolysis, a stable CO₂ supply source needs to be secured as a raw material. In addition, the liquid fuel synthesis system requires a large amount of energy in cooling for liquefying and capturing a produced liquid fuel gas and a condensable by-product gas.

A primary object of the present invention is to alleviate constraint on a place where a carbon dioxide-capturing system is installed, and/or to stably secure a CO₂ supply source and/or cooling energy in a liquid fuel production system.

### Solution to Problem

[1] According to an embodiment of the present invention, there is provided a liquid fuel production system including: a gas-adsorbing/desorbing portion configured to adsorb a predetermined gas A, and to desorb the gas A by being heated; a heating medium-supplying portion configured to supply, to the gas-adsorbing/desorbing portion, a heating medium for heating the gas-adsorbing/desorbing portion; a liquid fuel-synthesizing portion including a first gas flow path storing a catalyst that advances a conversion reaction from a raw material gas containing at least carbon dioxide and hydrogen to a liquid fuel, and a second gas flow path through which a temperature-controlling gas that controls a temperature of the first gas flow path is flowed, the liquid fuel-synthesizing portion being configured to flow a first outflow gas out of the first gas flow path, and to flow a second outflow gas out of the second gas flow path; and a heat exchanger configured to perform heat exchange between the first outflow gas and the heating medium to heat the heating medium, wherein the first outflow gas contains a condensable gas.
[2] In the liquid fuel production system according to the above-mentioned item [1], the gas-adsorbing/desorbing portion may be a carbon dioxide-adsorbing/desorbing portion, and the liquid fuel production system may further include a carbon dioxide-capturing portion, which includes the carbon dioxide-adsorbing/desorbing portion and is configured to capture a carbon dioxide-enriched gas from a carbon dioxide-containing gas.
[3] In the liquid fuel production system according to the above-mentioned item [2], the heating medium may contain the carbon dioxide-enriched gas.
[4] In the liquid fuel production system according to any one of the above-mentioned items [1] to [3], the liquid fuel-synthesizing portion may include a water vapor separation membrane, which is configured to cause water vapor to permeate therethrough, between the first gas flow path and the second gas flow path, and the first outflow gas may contain the liquid fuel.
[5] In the liquid fuel production system according to any one of the above-mentioned items [1] to [3], the liquid fuel-synthesizing portion may include a liquid fuel separation membrane, which is configured to cause at least the liquid fuel to permeate therethrough, between the first gas flow path and the second gas flow path, and the first outflow gas may contain water vapor that is a by-product of the conversion reaction.
[6] In the liquid fuel production system according to any one of the above-mentioned items [1] to [5], the temperature-controlling gas may contain at least one selected from hydrogen, carbon dioxide, carbon monoxide, nitrogen, and oxygen as a main component.
[7] In the liquid fuel production system according to any one of the above-mentioned items [2] to [6], the temperature-controlling gas may contain the carbon dioxide-enriched gas, and the liquid fuel production system may further include a temperature-controlling gas-circulating portion configured to capture the temperature-controlling gas from the liquid fuel-synthesizing portion, and to supply carbon dioxide derived from the carbon dioxide-enriched gas to the liquid fuel-synthesizing portion.
[8] In the liquid fuel production system according to any one of the above-mentioned items [2] to [7], the carbon dioxide-enriched gas may be supplied as a constituent component for the raw material gas from the carbon dioxide-capturing portion to the liquid fuel-synthesizing portion.
[9] According to an embodiment of the present invention, there is provided a method of producing a liquid fuel including: a step (I) of bringing a carbon dioxide-containing gas into contact with a carbon dioxide adsorbent to cause the carbon dioxide adsorbent to adsorb the carbon dioxide; a step (II) of desorbing the carbon dioxide from the carbon dioxide adsorbent through heating, followed by capture of a carbon dioxide-enriched gas; a step (III) of performing a conversion reaction from a raw material gas containing at least carbon dioxide and hydrogen to the liquid fuel with a liquid fuel-synthesizing portion including a first gas flow path storing a catalyst that advances the conversion reaction, and a second gas flow path through which a temperature-controlling gas that controls a temperature of the first gas flow path is flowed, the liquid fuel-synthesizing portion being configured to flow a first outflow gas out of the first gas flow path, and to flow a second outflow gas out of the second gas flow path; and a step (IV) of heating a heating medium for heating the carbon dioxide adsorbent in the step (II) through heat exchange using the first outflow gas as a heat medium, wherein the first outflow gas contains a condensable gas.
[10] In the production method according to the above-mentioned item [9], the temperature-controlling gas may contain at least one selected from hydrogen, carbon dioxide, carbon monoxide, nitrogen, and oxygen as a main component.
[11] In the production method according to the above-mentioned item [9] or [10], the temperature-controlling gas may contain the carbon dioxide-enriched gas, and carbon dioxide derived from the carbon dioxide-enriched gas may be captured from the second outflow gas and used as a constituent component for the raw material gas.
[12] In the production method according to any one of the above-mentioned items [9] to [11], the heating medium may contain the carbon dioxide-enriched gas.
[13] In the production method according to any one of the above-mentioned items [9] to [12], the liquid fuel-synthesizing portion may include a water vapor separation membrane, which is configured to cause water vapor to permeate therethrough, between the first gas flow path and the second gas flow path, and the first outflow gas may contain the liquid fuel.
[14] In the production method according to any one of the above-mentioned items [9] to [12], the liquid fuel-synthesizing portion may include a liquid fuel separation membrane, which is configured to cause at least the liquid fuel to permeate therethrough, between the first gas flow path and the second gas flow path, and the first outflow gas may contain water vapor that is a by-product of the conversion reaction.

### Advantageous Effects of Invention

According to the liquid fuel production system of the embodiment of the present invention, the gas containing the condensable gas, which is flowed out of the liquid fuel-synthesizing portion, may be used as a heat source for desorbing the gas A (e.g., CO₂). Thus, cooling energy for liquefying the condensable gas can be reduced. In addition, when the gas A is CO₂, a heat source for desorbing CO₂ can be secured, and hence constraint on a place where the carbon dioxide-capturing system (carbon dioxide-capturing portion) is installed can be alleviated. Further, a CO₂ source for liquid fuel synthesis can be secured by using CO₂ captured in the carbon dioxide-capturing system as a raw material for the liquid fuel synthesis.

### Brief Description of Drawings

FIG. 1A is a schematic configuration view of a liquid fuel production system according to one embodiment of the present invention.
FIG. 1B is a schematic configuration view of a liquid fuel production system according to another embodiment of the present invention.
FIG. 2 is a schematic configuration view of an example of a carbon dioxide-adsorbing/desorbing portion to be used in the liquid fuel production system according to the embodiment of the present invention.

### Description of Embodiments

Embodiments of the present invention are described below with reference to the drawings. However, the present invention is not limited to these embodiments. In addition, for clearer illustration, some widths, thicknesses, shapes, and the like of respective portions may be schematically illustrated in the drawings in comparison to the embodiments. However, the widths, the thicknesses, the shapes, and the like are each merely an example, and do not limit the understanding of the present invention.

### A. Liquid Fuel Production System

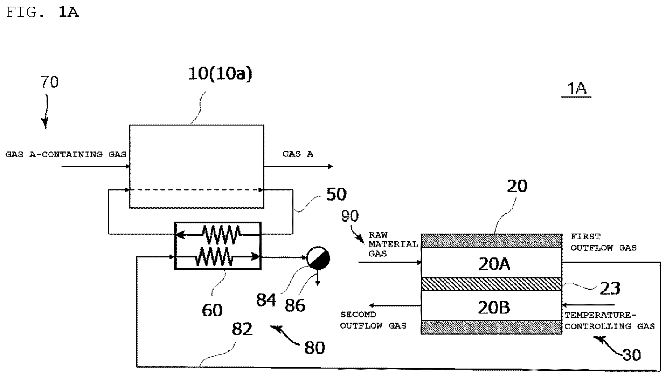

FIG. **1A** is a schematic configuration view of a liquid fuel production system according to one embodiment of the present invention.

A liquid fuel production system **1A** illustrated in FIG. **1A** includes:
a gas-adsorbing/desorbing portion **10a** configured to adsorb a predetermined gas A, and to desorb the gas A by being heated;
a heating medium-supplying portion **50** configured to supply, to the gas-adsorbing/desorbing portion **10a,** a heating medium for heating the gas-adsorbing/desorbing portion **10a;**
a liquid fuel-synthesizing portion **20** including a first gas flow path **20A** storing a catalyst that advances a conversion reaction from a raw material gas containing at least carbon dioxide and hydrogen to a liquid fuel, and a second gas flow path **20B** through which a temperature-controlling gas that controls a temperature of the first gas flow path **20A** is flowed, the liquid fuel-synthesizing portion **20** being configured to flow a first outflow gas out of the first gas flow path **20A,** and to flow a second outflow gas containing the temperature-controlling gas out of the second gas flow path **20B;** and
a heat exchanger **60** configured to perform heat exchange between the first outflow gas and the heating medium to heat the heating medium.

The first outflow gas contains a condensable gas.

According to the liquid fuel production system **1A,** the first outflow gas flowing out of the liquid fuel-synthesizing portion **20** is used as a heat source for desorbing the gas A in the gas-adsorbing/desorbing portion **10a.** Specifically, the heating medium for heating the gas-adsorbing/desorbing portion **10a** is heated through heat exchange by using the first outflow gas flowing out of the liquid fuel-synthesizing portion **20** as a heat source. Accordingly, when a carbon dioxide-adsorbing/desorbing portion, which adsorbs CO₂ and desorbs CO₂ by being heated, is applied as the gas-adsorbing/desorbing portion **10a,** a heat source for desorbing CO₂ can be secured, and hence constraint on a place where the carbon dioxide-capturing system (carbon dioxide-capturing portion) is installed can be alleviated. In addition, cooling energy for liquefying the condensable gas (e.g., the liquid fuel or water vapor) that has been flowed out of the liquid fuel-synthesizing portion **20** can be reduced. Herein, a significant reduction in temperature of the first outflow gas containing the condensable gas is prevented by condensing the condensable gas at the time of the heat exchange, and hence the outflow gas can function as an efficient heat medium. As described later, a CO₂-enriched gas may be used as the heating medium for heating the gas-adsorbing/desorbing portion **10a.** In addition, water (water vapor) may be used. Those media may be used alone, or in combination as a mixture thereof or in succession.

In addition, a CO₂ source in liquid fuel production may be secured by, for example, supplying the CO₂-enriched gas captured in the carbon dioxide-capturing portion as a constituent component for the raw material gas to the liquid fuel-synthesizing portion **20.**

In this description, the liquid fuel is a fuel that is in a liquid state at normal temperature and normal pressure or a fuel that can be liquefied under a normal-temperature pressurized state. Examples of the fuel that is in a liquid state at normal temperature and normal pressure include methanol, ethanol, a liquid fuel represented by CₙH₂₍ₘ₋₂ₙ₎ ("m" represents an integer of less than 90 and "n" represents an integer of less than 30), and a mixture thereof. Examples of the fuel that can be liquefied under a normal-temperature pressurized state include propane, butane, and a mixture thereof.

In the liquid fuel production system **1A,** a gas A-containing gas containing the predetermined gas A is supplied from a gas A-containing gas-supplying portion **70** to the gas-adsorbing/desorbing portion **10a.** In addition, the raw material gas for synthesizing the liquid fuel is supplied from a raw material gas-supplying portion **90** to the first gas flow path **20A** of the liquid fuel-synthesizing portion **20,** and the conversion reaction is advanced by the catalyst stored in the first gas flow path **20A** so as to be capable of being brought into contact with the raw material gas.

For example, the conversion reaction that synthesizes methanol through the catalytic hydrogenation of the raw material gas containing CO₂ and hydrogen in the presence of the catalyst is represented by the following reaction formula (1).

CO₂+3H₂ ↔ CH₃OH+H₂O (1)

The above-mentioned reaction is an equilibrium reaction, and hence, to increase both of its conversion ratio and reaction rate, the reaction is preferably performed under high temperature and high pressure (e.g., at 180°C or more and 1 MPa or more). Each of the liquid fuel and water that is a by-product is in a gas state at the time of its synthesis, and at least until the liquid fuel or the water flows out of the liquid fuel-synthesizing portion **20,** the liquid fuel or the water is maintained while being in a gas state.

In one embodiment, a partition **23** interposed between the first gas flow path **20A** and second gas flow path **20B** of the liquid fuel-synthesizing portion **20** includes a water vapor separation membrane that causes water vapor to permeate therethrough. Thus, the water vapor that is a by-product of the conversion reaction flows from the first gas flow path **20A** into the second gas flow path **20B.** The water vapor separation membrane is described later.

The temperature-controlling gas is supplied from a temperature-controlling gas-supplying portion **30** to the second gas flow path **20B.** The temperature-controlling gas is a gas that controls the temperature of the first gas flow path **20A,** and is, for example, a cooling medium that cools the first gas flow path **20A.** In this embodiment, the temperature-controlling gas also functions as a sweeping gas that sweeps the water vapor that has flowed into the second gas flow path **20B.**

A gas that has not permeated through the water vapor separation membrane, typically, for example, the liquid fuel or an unreacted raw material gas (remaining raw material gas) flows as the first outflow gas out of the first gas flow path **20A.** The first outflow gas is supplied to the heat exchanger **60** through a first outflow gas-capturing pipe **82,** and is subjected to the heat exchange with the heating medium supplied by the heating medium-supplying portion **50.** Thus, the heating medium is heated so as to be capable of heating the gas-adsorbing/desorbing portion **10a** to a predetermined temperature (temperature at which the gas A can be desorbed), and the liquid fuel incorporated into the first outflow gas is condensed, and is captured from a drain trap **84** and a liquefied product-capturing pipe **86.** The remaining raw material gas separated from the liquid fuel is reused as the raw material gas as required.

Meanwhile, the second outflow gas containing the water vapor and the temperature-controlling gas flows out of the second gas flow path **20B.** The second outflow gas is separated into water and the temperature-controlling gas by, for example, gas-liquid separation, and is removed or reused.

In another embodiment, the partition **23** interposed between the first gas flow path **20A** and second gas flow path **20B** of the liquid fuel-synthesizing portion **20** includes a liquid fuel separation membrane that causes the liquid fuel to permeate therethrough. Thus, the liquid fuel that is the target product of the conversion reaction flows from the first gas flow path 20A into the second gas flow path **20B.** A membrane described in, for example, JP 2020-23488 A may be used as the liquid fuel separation membrane. The liquid fuel separation membrane causes the liquid fuel to permeate therethrough with selectivity higher than that of the water vapor, and does not completely separate both the water vapor and the liquid fuel from each other.

The temperature-controlling gas, which also functions as a sweeping gas that sweeps the liquid fuel that has flowed into the second gas flow path **20B,** is supplied from the temperature-controlling gas-supplying portion **30** to the second gas flow path **20B.**

A gas that has not permeated through the liquid fuel separation membrane, typically, for example, the water vapor or an unreacted raw material gas (remaining raw material gas) flows as the first outflow gas out of the first gas flow path **20A.** The first outflow gas is supplied to the heat exchanger **60** through the first outflow gas-capturing pipe **82,** and is subjected to the heat exchange with the heating medium supplied by the heating medium-supplying portion **50.** Thus, the heating medium is heated so as to be capable of heating the gas-adsorbing/desorbing portion **10a** to a predetermined temperature (temperature at which the gas A can be desorbed), and the water vapor incorporated into the first outflow gas is condensed, and is captured from the drain trap **84** and the liquefied product-capturing pipe **86.** The remaining raw material gas separated from the water vapor is reused as the raw material gas as required.

Meanwhile, the second outflow gas containing the liquid fuel and the temperature-controlling gas flows out of the second gas flow path **20B.** The second outflow gas is separated into the liquid fuel and the temperature-controlling gas by, for example, gas-liquid separation, and each of the fuel and the gas is captured or reused.

In still another embodiment, the partition **23** interposed between the first gas flow path **20A** and second gas flow path **20B** of the liquid fuel-synthesizing portion **20** is a gas-impermeable partition that does not cause any gas to permeate therethrough. In this embodiment, the first outflow gas containing the liquid fuel, the water vapor, and the remaining raw material gas flows out of the first gas flow path **20A,** and the temperature-controlling gas flows as the second outflow gas out of the second gas flow path **20B.** The first outflow gas is supplied to the heat exchanger **60** through the first outflow gas-capturing pipe **82,** and is subjected to the heat exchange with the heating medium supplied by the heating medium-supplying portion **50.** Thus, the heating medium is heated so as to be capable of heating the gas-adsorbing/desorbing portion **10a** to a predetermined temperature (temperature at which the gas A can be desorbed), and the condensable gas (specifically, the liquid fuel and the water vapor) incorporated into the first outflow gas is condensed, and is captured from the drain trap **84** and the liquefied product-capturing pipe **86.**

In the illustrated example, the first gas flow path **20A** and the second gas flow path **20B** are vertically divided through the partition **23.** However, the second gas flow path **20B** only needs to be arranged so as to be capable of controlling the temperature of the first gas flow path **20A.** For example, the liquid fuel-synthesizing portion **20** may have such a double tube structure that one of an inner tube or an outer tube is the first gas flow path, and the other thereof is the second gas flow path.

In one embodiment, the temperature-controlling gas contains at least one selected from hydrogen, carbon dioxide, carbon monoxide, nitrogen, and oxygen as a main component. The phrase "contains as a main component" as used herein means that the gas contains the component at the highest content.

### A-1. One Example of Liquid Fuel Production System

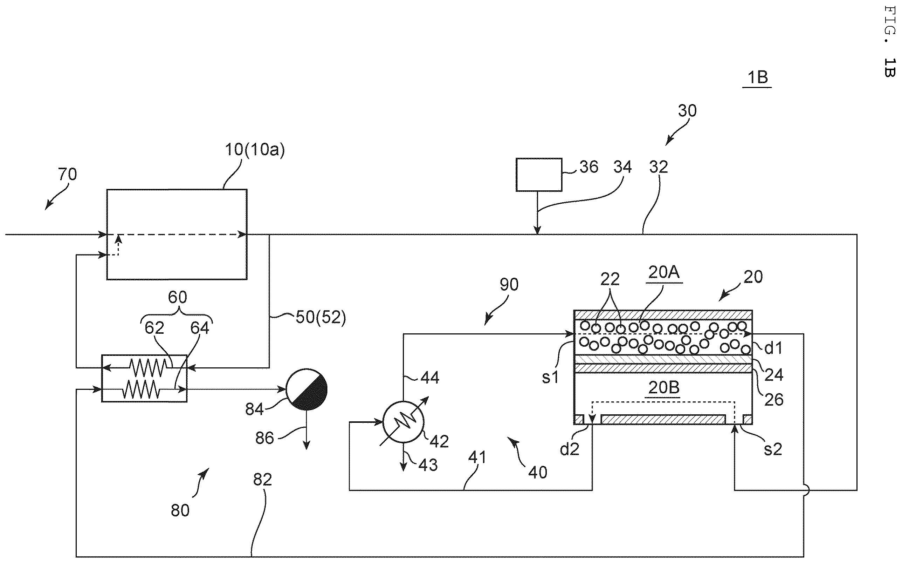

A liquid fuel production system **1B** illustrated in FIG. **1B** is described below as an example of the embodiment in which the liquid fuel-synthesizing portion **20** includes the water vapor separation membrane between the first gas flow path **20A** and the second gas flow path **20B.** The liquid fuel production system **1B** includes the liquid fuel-synthesizing portion including the water vapor separation membrane, and the temperature-controlling gas supplied to the liquid fuel-synthesizing portion can also function as a sweeping gas. Accordingly, in the following description, the temperature-controlling gas may be referred to as "sweeping gas."

The liquid fuel production system **1B** illustrated in FIG. **1B** includes:
a carbon dioxide-capturing portion **10** configured to capture a CO₂-enriched gas from a CO₂-containing gas, the carbon dioxide-capturing portion **10** including the carbon dioxide-adsorbing/desorbing portion **10a** configured to adsorb CO₂, and to desorb CO₂ by being heated;
the liquid fuel-synthesizing portion **20** including the first gas flow path **20A** storing a catalyst that advances a conversion reaction from a raw material gas containing at least CO₂ and hydrogen to a liquid fuel, and the second gas flow path **20B** through which a sweeping gas that controls the temperature of the first gas flow path **20A** is flowed, the liquid fuel-synthesizing portion **20** being configured to flow a first outflow gas out of the first gas flow path **20A,** and to flow a second outflow gas out of the second gas flow path **20B;**
the raw material gas-supplying portion **90** that supplies the raw material gas to the liquid fuel-synthesizing portion **20;**
the sweeping gas-supplying portion **30** that supplies the CO₂-enriched gas as a constituent component for the sweeping gas to the liquid fuel-synthesizing portion **20;**
a sweeping gas-circulating portion **40** configured to capture the sweeping gas from the liquid fuel-synthesizing portion **20,** and to supply CO₂ derived from the CO₂-enriched gas to the raw material gas-supplying portion **90;**
the heating medium-supplying portion **50** configured to supply the CO₂-enriched gas as a heating medium for heating a carbon dioxide adsorbent to the carbon dioxide-adsorbing/desorbing portion **10a;** and
the heat exchanger **60** configured to perform heat exchange between the first outflow gas and the heating medium to heat the heating medium.

The CO₂-containing gas is supplied from the carbon dioxide-containing gas-supplying portion **70** to the carbon dioxide-capturing portion **10.**

The respective constituent portions are more specifically described below with reference to the liquid fuel production system **1B.**

### A-1-1. Carbon Dioxide-capturing Portion

The carbon dioxide-capturing portion **10** includes the carbon dioxide-adsorbing/desorbing portion **10a.** The carbon dioxide-capturing portion **10** may further include, for example, a pump or a carbon dioxide-enriched gas-storing portion. The carbon dioxide-adsorbing/desorbing portion **10a** stores, for example, the carbon dioxide adsorbent, which adsorbs CO₂ by being brought into contact with the CO₂-containing gas, and which desorbs CO₂ by being heated. With such configuration, the carbon dioxide-capturing portion **10** supplies the CO₂-containing gas to the carbon dioxide-adsorbing/desorbing portion **10a,** and causes the carbon dioxide adsorbent to adsorb CO₂. Next, the portion heats the carbon dioxide adsorbent to cause the adsorbent to desorb CO₂, and sucks the desorbed CO₂ with the pump. Thus, the portion can capture the CO₂-enriched gas containing CO₂ at a concentration higher than that of the CO₂-containing gas supplied to the carbon dioxide-adsorbing/desorbing portion **10a.**

The carbon dioxide-adsorbing/desorbing portion **10a** has, for example, a configuration in which a gas can flow into and out of the portion, and the carbon dioxide adsorbent is arranged so as to be capable of being brought into contact with the CO₂-containing gas that has flowed thereinto. The carbon dioxide adsorbent is preferably carried by any appropriate base material. In one embodiment, the carbon dioxide-adsorbing/desorbing portion **10a** includes: the base material; and a carbon dioxide-adsorbing layer, which is arranged on the surface of the base material and contains the carbon dioxide adsorbent.

The structure of the base material is not particularly limited, and examples thereof include: a filter structure, such as a monolith shape or a filter cloth; and a pellet structure. The monolith shape means a shape having a plurality of cells penetrating the shape in its lengthwise direction, and is a concept including a honeycomb shape. In one embodiment, the base material is a honeycomb-shaped base material having a plurality of cells. The sectional shape of each of the cells (sectional shape in a direction perpendicular to the lengthwise direction of the honeycomb-shaped base material) is, for example, a triangle, a quadrangle, a pentagon, a polygon that is hexagonal or more, a circular shape, or an elliptical shape.

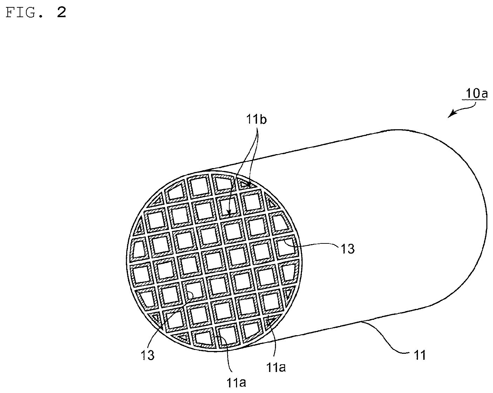

In one embodiment, as illustrated in FIG. 2, the carbon dioxide-adsorbing/desorbing portion 10a includes: a honeycomb-shaped base material 11 having a plurality of cells 11b defined by partitions 11a; and carbon dioxide-adsorbing layers **13** formed on the inner surfaces of the cells **11b** (in other words, the surfaces of the partitions **11a**). The sectional shape of each of the cells in the honeycomb-shaped base material of the illustrated example is a quadrangle, and the CO₂-containing gas is supplied to a space in the section where the carbon dioxide-adsorbing layer **13** is not formed.

Typical examples of a material for forming the base material include ceramics. Examples of the ceramics include silicon carbide, a silicon-silicon carbide-based composite material, cordierite, mullite, alumina, silicon nitride, spinel, a silicon carbide-cordierite-based composite material, lithium aluminum silicate, and aluminum titanate. The materials for forming the base material may be used alone or in combination thereof.

The carbon dioxide-adsorbing layer is not particularly limited as long as the layer is arranged on the surface of the base material so as to be capable of being brought into contact with the CO₂-containing gas. The carbon dioxide-adsorbing layer may contain substantially only the carbon dioxide adsorbent, or may contain any other component except the carbon dioxide adsorbent (e.g., a porous carrier).

Any appropriate compound that can adsorb and desorb CO₂ may be adopted as the carbon dioxide adsorbent. Examples of the carbon dioxide adsorbent include: nitrogen-containing compounds to be described later; alkali compounds, such as sodium hydroxide and potassium hydroxide; carbonates, such as calcium carbonate and potassium carbonate; hydrogen carbonates, such as calcium hydrogen carbonate and potassium hydrogen carbonate; metal organic frameworks (MOFs), such as MOF-74, MOF-200, and MOF-210; zeolite; activated carbon; nitrogen-doped carbon; and ion liquids.

Of the carbon dioxide adsorbents, for example, nitrogen-containing compounds are more preferred. More specific examples of the nitrogen-containing compounds include: amine compounds, such as monoethanolamine, diethanolamine, triethanolamine, N-(3-aminopropyl)diethanolamine, aminopropyltrimethoxysilane, and polyvinylamine; imine compounds, such as polyethylenimine and polyethylenimine-trimethoxysilane; amide compounds such as polyamidoamine; piperazine compounds such as 1-(2-hydroxyethyl)piperazine; and aminosilane coupling agents, such as 3-aminopropyltriethoxysilane and N-(2-aminoethyl)-3-aminopropyltrimethoxysilane. The adsorbents may be used alone or in combination thereof.

The carbon dioxide-adsorbing layer may be produced by, for example, the following method. First, the carbon dioxide adsorbent and any appropriate other component are dissolved or dispersed in a solvent to prepare a carbon dioxide-adsorbing layer-forming liquid. Examples of the solvent include water, alcohols, diols, and combinations thereof. Next, the carbon dioxide-adsorbing layer-forming liquid is applied onto the base material, and then the coating film is dried, and as required, sintered to form the carbon dioxide-adsorbing layer.

### A-1-2. Liquid Fuel-synthesizing Portion

The liquid fuel-synthesizing portion 20 is a so-called membrane reactor for converting the raw material gas into the liquid fuel. The shape of the liquid fuel-synthesizing portion **20** is not particularly limited, but may be set to, for example, a monolith shape, a flat-plate shape, a tubular shape, a cylindrical shape, a columnar shape, or a polygonal columnar shape.

The liquid fuel-synthesizing portion **20** includes: a catalyst **22** that advances the conversion reaction from the raw material gas containing at least CO₂ and hydrogen to the liquid fuel; a water vapor separation membrane **24;** a non-permeation-side space **20A;** and a permeation-side space **20B.** In the liquid fuel-synthesizing portion **20,** a first supply port **s1** and a first discharge port **d1** that communicate to each other through the non-permeation-side space **20A,** and a second supply port **s2** and a second discharge port **d2** that communicate to each other through the permeation-side space **20B** are arranged.

The non-permeation-side space **20A** is a space on the non-permeation side of the water vapor separation membrane **24.** The raw material gas is supplied from the raw material gas-supplying portion **90** to the non-permeation-side space **20A** through the first supply port **s1**. The liquid fuel synthesized in the catalyst **22** and the remaining raw material gas flow as the first outflow gas out of the non-permeation-side space **20A** to a first outflow gas-capturing portion **80** through the first discharge port **d1**. That is, the non-permeation-side space **20A** is the first gas flow path.

The permeation-side space **20B** is a space on the permeation side of the water vapor separation membrane **24.** As described later, water vapor and part of hydrogen in the raw material gas permeate through the water vapor separation membrane **24,** and hence the water vapor and hydrogen flow into the permeation-side space **20B.** In addition, the sweeping gas is supplied from the sweeping gas-supplying portion **30** to the permeation-side space **20B** through the second supply port **s2.** The water vapor, hydrogen, and the sweeping gas flow as the second outflow gas out of the permeation-side space **20B** to the sweeping gas-circulating portion **40** through the second discharge port **d2.** That is, the permeation-side space **20B** is the second gas flow path.

The catalyst **22** advances the conversion reaction from the raw material gas to the liquid fuel. The catalyst is arranged in the non-permeation-side space **20A.** Although the catalyst is preferably filled into the non-permeation-side space **20A,** the catalyst may be arranged in a layer shape or an island shape on the surface of the water vapor separation membrane **24.** When the catalyst has a particle shape like the illustrated example, the particle diameters (diameters) of catalyst particles may each be set to, for example, 0.5 mm or more and 10 mm or less. Each of the catalyst particles may be formed only of the catalyst, or may have a configuration in which the catalyst is carried by a carrier particle. The carrier particle is preferably a porous particle.

Any catalyst suitable for a conversion reaction to a desired liquid fuel may be used as the catalyst. Specifically, metal catalysts (e.g., copper and palladium), oxide catalysts (e.g., zinc oxide, zirconia, and gallium oxide), and catalysts obtained by compositing these catalysts (e.g., copper-zinc oxide, copper-zinc oxide-alumina, copper-zinc oxide-chromium oxide-alumina, copper-cobalt-titania, and catalysts obtained by modifying these catalysts with palladium) may each be used.

The water vapor separation membrane **24** causes water vapor, which is a by-product of the conversion reaction from the raw material gas to the liquid fuel, to permeate therethrough. Thus, the reaction equilibrium of the formula (1) can be shifted to a product side through utilization of an equilibrium shift effect.

The molecular diameter (0.26 nm) of water is close to the molecular diameter (0.296 nm) of hydrogen. Accordingly, not only the water vapor that is a by-product of the conversion reaction but also part of hydrogen in the raw material gas can permeate through the water vapor separation membrane **24.**

The water vapor separation membrane **24** preferably has a water vapor permeability coefficient of 100 nmol/(s·Pa·m²) or more. The water vapor permeability coefficient may be determined by an existing method (see Ind. Eng. Chem. Res., 40, 163-175 (2001)).

The water vapor separation membrane **24** preferably has a separation coefficient of 100 or more. As the separation coefficient becomes larger, the membrane more easily causes the water vapor to permeate therethrough, and more hardly causes components except the water vapor (e.g., hydrogen, carbon dioxide, and the liquid fuel) to permeate therethrough. The separation coefficient may be determined by an existing method (see Fig. 1 of "Separation and Purification Technology 239 (2020) 116533").

Any appropriate membrane may be used as the water vapor separation membrane **24.** In one embodiment, an inorganic membrane may be used as the water vapor separation membrane **24.** The inorganic membrane is preferred because the membrane has heat resistance, pressure resistance, water vapor resistance, and the like. Examples of the inorganic membrane include a zeolite membrane, a silica membrane, an alumina membrane, and a composite membrane thereof. For example, an LTA zeolite membrane in which the molar ratio (Si/Al) of a silicon element (Si) to an aluminum element (Al) is 1.0 or more and 3.0 or less is suitable because the membrane is excellent in water vapor permeability.

The zeolite membrane to be used as the water vapor separation membrane **24** may be obtained by a production method described in JP 2004-66188 A. In addition, the silica membrane to be used as the water vapor separation membrane **24** may be obtained by a production method described in WO 2008/050812 A1.

In the illustrated example, the water vapor separation membrane **24** is supported by a porous support **26.**

The porous support **26** includes a porous material. For example, a ceramic material, a metal material, and a resin material may each be used as the porous material, and the ceramic material is particularly suitable. For example, alumina (Al₂O₃), titania (TiO₂), mullite (Al₂O₃·SiO₂), ceramic fragments, and cordierite (Mg₂Al₄Si₅O₁₈) may each be used as an aggregate for the ceramic material, and alumina is suitable in consideration of ease of availability, green body stability, and corrosion resistance. At least one of titania, mullite, easily sinterable alumina, silica, glass frit, a clay mineral, or easily sinterable cordierite may be used as an inorganic binder for the ceramic material. The ceramic material may be free of any inorganic binder.

The average pore diameter of the porous support may be set to 5 µm or more and 25 µm or less. The average pore diameter of the porous support may be measured by a mercury intrusion method. The porosity of the porous support may be set to 25% or more and 50% or less. The average particle diameter of the porous material for forming the porous support may be set to 1 µm or more and 100 µm or less. In this embodiment, the average particle diameter is the arithmetic average value of the maximum diameters of 30 measurement object particles (randomly selected) measured by sectional microstructure observation with a scanning electron microscope (SEM).

### A-1-3. Carbon Dioxide-containing Gas-supplying Portion

The carbon dioxide-containing gas-supplying portion **70** optionally includes a blower or the like, and supplies the CO₂-containing gas from air or various facilities, such as a factory and a commercial facility, to the carbon dioxide-capturing portion **10** (more specifically, the carbon dioxide-adsorbing/desorbing portion **10a**).

### A-1-4. Sweeping Gas-supplying Portion

The sweeping gas-supplying portion **30** is configured to supply the CO₂-enriched gas as a constituent component for the sweeping gas from the carbon dioxide-capturing portion **10** to the liquid fuel-synthesizing portion **20.** The sweeping gas-supplying portion **30** includes: a carbon dioxide-enriched gas-supplying pipe **32;** a hydrogen-supplying pipe **34;** and a hydrogen-producing portion **36.** One end of the carbon dioxide-enriched gas-supplying pipe **32** is connected to the carbon dioxide-capturing portion **10,** and the other end thereof is connected to the second supply port **s2** of the liquid fuel-synthesizing portion **20.** One end of the hydrogen-supplying pipe **34** is connected to the hydrogen-producing portion **36,** and the other end thereof is connected to the carbon dioxide-enriched gas-supplying pipe **32.** The hydrogen-producing portion **36** is, for example, a hydrogen-producing facility utilizing a water electrolysis technology. The CO₂-enriched gas supplied from the carbon dioxide-capturing portion **10** to the liquid fuel-synthesizing portion **20** through the carbon dioxide-enriched gas-supplying pipe **32** is mixed with hydrogen supplied from the hydrogen-producing portion **36.** Thus, the sweeping gas containing the CO₂-enriched gas and hydrogen is produced. The sweeping gas is heated to a desired temperature (e.g., 150°C or more and 350°C or less), and is then supplied to the second supply port **s2** of the liquid fuel-synthesizing portion **20.**

In one embodiment, the sweeping gas contains hydrogen as a main component. The phrase "contains hydrogen as a main component" as used herein means that the content of hydrogen out of the gases incorporated into the sweeping gas is highest.

The concentration of CO₂ in the sweeping gas is, for example, 10 vol% or more and 40 vol% or less, preferably 20 vol% or more and 30 vol% or less. The concentration of hydrogen in the sweeping gas is, for example, 60 vol% or more and 90 vol% or less, preferably 70 vol% or more and 80 vol% or less. When the sweeping gas contains CO₂ and hydrogen at the above-mentioned ratios, the gases (the remaining raw material gas and the sweeping gas) captured from the liquid fuel-synthesizing portion can be suitably cyclically used as the raw material gas.

### A-1-5. First Outflow Gas-capturing Portion, Heating Medium-supplying Portion, and Heat Exchanger

The first outflow gas-capturing portion **80** includes: the first outflow gas-capturing pipe **82;** the drain trap **84;** and the liquefied product-capturing pipe **86.** One end of the first outflow gas-capturing pipe **82** is connected to the first discharge port **d1** of the liquid fuel-synthesizing portion **20,** and the other end thereof is connected to the drain trap **84.** The first outflow gas, which contains the liquid fuel and the remaining raw material gas that have not permeated through the water vapor separation membrane **24,** flows into the first outflow gas-capturing pipe **82** through the first discharge port **d1**.

The heating medium-supplying portion **50** includes a heating medium-supplying pipe **52,** and supplies the CO₂-enriched gas captured in the carbon dioxide-capturing portion **10** as the heating medium for heating the carbon dioxide adsorbent to the carbon dioxide-adsorbing/desorbing portion **10a.** The CO₂-enriched gas captured in the carbon dioxide-capturing portion **10** is supplied to the carbon dioxide-adsorbing/desorbing portion **10a** again through the heating medium-supplying pipe **52,** and captures the desorbed CO₂. Thus, the gas is captured again as a CO₂-enriched gas, which contains CO₂ at a concentration higher than that of the CO₂-enriched gas supplied as the heating medium, in the carbon dioxide-capturing portion **10.** Specifically, the CO₂-enriched gas serving as the heating medium flows through the same flow path as that of the CO₂-containing gas in the carbon dioxide-adsorbing/desorbing portion **10a** to heat the carbon dioxide adsorbent. Thus, the gas may be captured as a higher concentration CO₂-enriched gas further containing the desorbed CO₂ in the carbon dioxide-capturing portion **10.** In, for example, the carbon dioxide-adsorbing/desorbing portion **10a** illustrated in FIG. **2****,** the space in the section of each of the cells **11b** in the honeycomb-shaped base material **11** where the carbon dioxide-adsorbing layer **13** is not formed may be used as a flow path shared by the CO₂-containing gas and the CO₂-enriched gas serving as the heating medium. When the CO₂-enriched gas is circulated as described above, a CO₂-enriched gas containing CO₂ at a high concentration (e.g., having a CO₂ concentration of 50 vol% or more) can be suitably obtained. The configuration of the heating medium-supplying pipe **52** is not limited to the illustrated example, and the pipe may be configured as follows: the pipe is connected to the carbon dioxide-containing gas-supplying portion **70** to supply the CO₂-enriched gas to the carbon dioxide-adsorbing/desorbing portion **10a.** In addition, the heating medium-supplying portion may further include a second heating medium-supplying pipe that supplies another heating medium except the CO₂-enriched gas (e.g., water vapor) to the carbon dioxide-adsorbing/desorbing portion, though the pipe is not shown. In this case, the second heating medium-supplying pipe may be configured as follows: the pipe is connected to another heating medium-storing portion, and hence the other heating medium is supplied to the carbon dioxide-adsorbing/desorbing portion **10a** while being in a heated state through heat exchange with the heat exchanger **60.**

The heat exchanger **60** is interposed between the heating medium-supplying portion **50** and the first outflow gas-capturing portion **80.** Specifically, the heat exchanger **60** includes a first flow path **62** interposed in the heating medium-supplying pipe **52** and a second flow path **64** interposed in the first outflow gas-capturing pipe **82,** and is configured so that heat exchange can be performed between a first fluid flowing through the first flow path **62** and a second fluid flowing through the second flow path **64.**

According to the above-mentioned configuration, the heat exchange is performed between the heating medium (specifically, the CO₂-enriched gas) flowing through the first flow path **62** and the first outflow gas flowing through the second flow path **64,** and hence the heating medium is heated. In addition, when a condensable gas such as the liquid fuel incorporated into the first outflow gas is condensed at the time of the heat exchange, condensation heat can be utilized in the heat exchange, and the utilization can contribute to a further improvement in thermal efficiency.

The temperature of the heating medium before the heat exchange is, for example, 5°C or more and 100°C or less, preferably 10°C or more and 80°C or less. The temperature of the heating medium after the heat exchange is, for example, 60°C or more and 200°C or less, preferably 90°C or more and 160°C or less. The temperature of the first outflow gas before the heat exchange is, for example, 150°C or more and 350°C or less, preferably 200°C or more and 300°C or less. The temperature of the first outflow gas after the heat exchange is, for example, 25°C or more and 250°C or less, preferably 50°C or more and 200°C or less.

The concentration of the condensable gas in the first outflow gas before the heat exchange is, for example, 5 vol% or more and 50 vol% or less, preferably 10 vol% or more and 40 vol% or less. When the concentration of the condensable gas in the first outflow gas falls within the above-mentioned ranges, the heat exchange can be performed with higher efficiency.

The drain trap **84** is arranged on the downstream side of the heat exchanger **60.** The drain trap **84** separates a liquid (typically, the liquid fuel) condensed in the heat exchanger **60** and a gas from each other. The separated liquid is captured from the liquefied product-capturing pipe **86.**

The gas (remaining raw material gas) separated by the drain trap **84** is resupplied to the raw material gas-supplying portion **90** as required.

### A-1-6. Sweeping Gas-circulating Portion

The sweeping gas-circulating portion **40** includes: a second outflow gas-capturing pipe **41;** a condenser **42;** a liquefied product-capturing pipe **43;** and a captured gas-supplying pipe **44.** The second outflow gas-capturing pipe **41** is connected to the second discharge port **d2** of the liquid fuel-synthesizing portion **20** and the condenser **42,** and supplies the second outflow gas, which contains the water vapor and hydrogen that have permeated through the water vapor separation membrane **24,** and the sweeping gas, from the liquid fuel-synthesizing portion **20** to the condenser **42.**

The condenser **42** liquefies a condensable gas (typically, the water vapor) in the second outflow gas to be separated from the sweeping gas and hydrogen. A liquefied product (typically, water) is captured from the liquefied product-capturing pipe **43.** The separated gas (captured gas) is supplied to the raw material gas-supplying portion **90** through the captured gas-supplying pipe **44.** The captured gas contains the sweeping gas and hydrogen that has permeated through the water vapor separation membrane **24.**

### A-1-7. Raw Material Gas-supplying Portion

The raw material gas-supplying portion **90** includes a pressure-boosting portion or the like, and supplies the raw material gas at a desired pressure and a desired temperature to the liquid fuel-synthesizing portion **20.** In the illustrated example, the captured gas is supplied as the raw material gas to the liquid fuel-synthesizing portion **20.** However, the raw material gas may be prepared by being further mixed with, for example, the remaining raw material gas captured from the first outflow gas, hydrogen or carbon dioxide that is separately prepared, or a mixed gas thereof as required. Alternatively, the raw material gas may be completely free of the captured gas.

The embodiment in which the liquid fuel-synthesizing portion **20** includes the water vapor separation membrane between the first gas flow path **20A** and the second gas flow path **20B** has been described above with reference to FIG. **1B****.** However, the above-mentioned description may be similarly applied to the embodiment in which the liquid fuel-synthesizing portion includes the liquid fuel separation membrane between the first gas flow path and the second gas flow path. Specifically, the first outflow gas that has flowed out of the non-permeation-side space **20A,** the gas containing the water vapor and the remaining raw material gas, is used in the heat exchange with the heating medium, and a liquid (typically, water) produced by condensation at the time of the heat exchange and a gas are separated from each other. The separated gas (typically, the remaining raw material gas) may be supplied to the raw material gas-supplying portion to be reused as a constituent component for the raw material gas. In addition, the second outflow gas contains the liquid fuel and the sweeping gas (preferably a sweeping gas containing a CO₂-enriched gas), and the fuel and the gas are separated from each other by gas-liquid separation. The liquid fuel is captured from the liquefied product-capturing pipe, and the sweeping gas may be supplied to the raw material gas-supplying portion to be utilized as a constituent component for the raw material gas.

### A-2. Modification Example 1

In the embodiment illustrated in FIG. **1B****,** the liquid fuel-synthesizing portion has been a membrane reactor. However, the portion is not limited to the configuration. For example, the liquid fuel-synthesizing portion may be configured as follows: the portion includes a catalyst portion containing the catalyst and a separating portion, which includes the water vapor separation membrane, the non-permeation-side space, and the permeation-side space, as separate reactors in the stated order from its upstream side; a liquid fuel gas, a by-product gas (water vapor), and the remaining raw material gas that have flowed out of the catalyst portion flow into the non-permeation-side space of the separating portion; the by-product gas and hydrogen that have permeated through the water vapor separation membrane flow as the second outflow gas out of the permeation-side space of the separating portion together with the sweeping gas; and the liquid fuel and the remaining raw material gas that have not permeated through the water vapor separation membrane flow as the first outflow gas out of the non-permeation-side space thereof. In Modification Example 1, the gases that have flowed out of the catalyst portion flow as they are into the separating portion. Accordingly, the non-permeation-side space of the separating portion is regarded as forming the first gas flow path integrally with the catalyst portion, and the permeation-side space of the separating portion is regarded as the second gas flow path.

### A-3. Modification Example 2

In the embodiment illustrated in FIG. 1B, the flow directions of the raw material gas and the sweeping gas in the side view of the water vapor separation membrane **24** are opposite to each other. However, those directions may be identical to each other.

### A-4. Modification Example 3

The liquid fuel-synthesizing portion may further include a buffer layer between the catalyst **22** and the water vapor separation membrane **24.** The buffer layer is arranged for preventing the catalyst in the catalyst **22** from being brought into direct contact with the water vapor separation membrane **24.** When the catalyst and the water vapor separation membrane **24** are physically isolated from each other with the buffer layer, even in the case where the catalyst is heated by reaction heat, the occurrence of a crack in the water vapor separation membrane **24** starting from its point of contact with the catalyst can be suppressed.

### B. Method of producing Liquid Fuel

A method of producing a liquid fuel according to an embodiment of the present invention includes:
a step (I) of bringing a CO₂-containing gas into contact with a carbon dioxide adsorbent to cause the carbon dioxide adsorbent to adsorb the carbon dioxide;
a step (II) of desorbing the carbon dioxide from the carbon dioxide adsorbent through heating, followed by capture of a carbon dioxide-enriched gas;
a step (III) of performing a conversion reaction from a raw material gas containing at least carbon dioxide and hydrogen to the liquid fuel with a liquid fuel-synthesizing portion including a first gas flow path storing a catalyst that advances the conversion reaction, and a second gas flow path through which a temperature-controlling gas that controls a temperature of the first gas flow path is flowed, the liquid fuel-synthesizing portion being configured to flow a first outflow gas out of the first gas flow path, and to flow a second outflow gas out of the second gas flow path; and
a step (IV) of heating a heating medium for heating the carbon dioxide adsorbent in the step (II) through heat exchange using the first outflow gas as a heat medium.

The first outflow gas contains a condensable gas.

The heat exchange is preferably performed by utilizing the condensation heat of the condensable gas.

The method of producing a liquid fuel according to the embodiment of the present invention may be suitably performed by using the liquid fuel production system described in the section A.

In one embodiment, the temperature-controlling gas contains at least one selected from hydrogen, carbon dioxide, carbon monoxide, nitrogen, and oxygen as a main component.

In one embodiment, the temperature-controlling gas contains the CO₂-enriched gas, and carbon dioxide derived from the CO₂-enriched gas is captured from the second outflow gas and used as a constituent component for the raw material gas.

In one embodiment, the heating medium contains the CO₂-enriched gas.

### B-1. Step (I)

In the step (I), the CO₂-containing gas is brought into contact with the carbon dioxide adsorbent to cause the carbon dioxide adsorbent to adsorb CO₂. The concentration of CO₂ in the CO₂-containing gas is, for example, 0.01 vol% or more and 2 vol% or less. The temperature of the CO₂-containing gas is, for example, 0°C or more and 40°C or less. The pressure of the CO₂-containing gas is, for example, 0.3×10⁵ Pa or more and 2.0×10⁵ Pa or less. The relative humidity RH of the CO₂-containing gas is, for example, 10%RH or more and 60%RH or less. The time period for which the step (I) is performed is, for example, 15 minutes or more and 3 hours or less. The flow velocity of the CO₂-containing gas in the step (I) is, for example, 0.5 m/sec or more and 5 m/sec or less.

The adsorption ratio of CO₂ in the step (I) (=(concentration of CO₂ in gas before contact with carbon dioxide adsorbent-concentration of CO₂ in gas after contact with carbon dioxide adsorbent) /concentration of CO₂ in gas before contact with carbon dioxide adsorbent×100) is, for example, 60% or more, preferably 75% or more, more preferably 80% or more, and is, for example, 90% or less.

### B-2. Step (II)

In the step (II), CO₂ is desorbed from the carbon dioxide adsorbent through the heating, and the CO₂-enriched gas is captured. The CO₂-enriched gas is captured by, for example, heating the carbon dioxide adsorbent that has adsorbed CO₂ in the step (I) to desorb CO₂ from the carbon dioxide adsorbent, and sucking CO₂ desorbed (released) from the carbon dioxide adsorbent with a pump or the like.

The heating temperature of the step (II) is, for example, more than 40°C, preferably 70°C or more, and is, for example, 200°C or less, preferably 110°C or less. The heating time of the step (II) is, for example, 1 minute or more and 1 hour or less.

The CO₂-enriched gas contains CO₂ at a concentration higher than that of the CO₂-containing gas. The concentration of CO₂ in the CO₂-enriched gas is, for example, 50 vol% or more, preferably 60 vol% or more, more preferably 80 vol% or more, and is, for example, 100 vol% or less. The CO₂-enriched gas having such CO₂ concentration is suitable as a constituent component for the sweeping gas (finally, the raw material gas).

In one embodiment, the heating of the carbon dioxide adsorbent is performed with the heating medium containing the CO₂-enriched gas, and may be performed by using, for example, the CO₂-enriched gas as a heating medium. Specifically, the captured CO₂-enriched gas is heated to the temperature at which the carbon dioxide adsorbent can desorb CO₂, and the carbon dioxide adsorbent is heated with the CO₂-enriched gas. According to, for example, the liquid fuel production system **1B** illustrated in FIG. **1B****,** the CO₂-enriched gas that has been flowed out of the carbon dioxide-capturing portion **10** is supplied again as a heating medium to the carbon dioxide-adsorbing/desorbing portion **10a,** which has adsorbed CO₂, while the gas is in the state of being heated in the heat exchanger **60,** and the gas captures the desorbed CO₂. Thus, the gas is captured as a CO₂-enriched gas, which contains CO₂ at a concentration higher than that at the time of its supply as the heating medium, in the carbon dioxide-capturing portion **10.** When the circulation of the CO₂-enriched gas is repeated as described above, a CO₂-enriched gas containing CO₂ at a higher concentration can be captured.

### B-3. Step (III)

In the step (III), the conversion reaction from the raw material gas containing at least carbon dioxide and hydrogen to the liquid fuel is performed with the liquid fuel-synthesizing portion including the first gas flow path storing the catalyst that advances the conversion reaction, and the second gas flow path through which the temperature-controlling gas that controls the temperature of the first gas flow path is flowed, the liquid fuel-synthesizing portion being configured to flow the first outflow gas out of the first gas flow path, and to flow the second outflow gas out of the second gas flow path. Specifically, the raw material gas is caused to flow into the first gas flow path, and as represented by the reaction formula (1), the raw material gas containing CO₂ and hydrogen is subjected to catalytic hydrogenation in the presence of the catalyst to synthesize methanol. In addition, the temperature-controlling gas is caused to flow into the second gas flow path.

The liquid fuel-synthesizing portion includes, for example, a separation membrane, which causes one of condensable gases, that is, the liquid fuel and water vapor, to permeate therethrough to separate the gas from the other, between the first gas flow path and the second gas flow path. A water vapor separation membrane or a liquid fuel separation membrane may be used as such separation membrane. When the liquid fuel-synthesizing portion includes the water vapor separation membrane, the first outflow gas containing the liquid fuel and the second outflow gas containing the water vapor are flowed out of the liquid fuel-synthesizing portion. When the liquid fuel-synthesizing portion includes the liquid fuel separation membrane, the first outflow gas containing the water vapor and the second outflow gas containing the liquid fuel are flowed out of the liquid fuel-synthesizing portion.

The concentration of CO₂ in the raw material gas is, for example, 10 vol% or more and 40 vol% or less, preferably 20 vol% or more and 30 vol% or less. The concentration of hydrogen in the raw material gas is, for example, 60 vol% or more and 90 vol% or less, preferably 70 vol% or more and 80 vol% or less.

The reaction temperature of the step (III) is, for example, 180°C or more, preferably 200°C or more and 350°C or less. The pressure thereof is, for example, 1 MPa or more, preferably 2 MPa or more and 6 MPa or less. The flow velocity of the raw material gas is appropriately adjusted in accordance with, for example, a catalyst characteristic and a reactor form, and is, for example, 1,000/h or more and 50,000/h or less, preferably 2,000/h or more and 20,000/h or less, more preferably 3,000/h or more and 12,000/h or less in terms of gas hourly space velocity (GHSV) in a standard state (0°C and 1 atm).

### B-4. Step (IV)

In the step (IV), the heating medium for heating the carbon dioxide adsorbent in the step (II) is heated through the heat exchange using the first outflow gas as the heat medium. When the first outflow gas obtained in the step (III) is used as a heat source for desorbing CO₂, cooling energy for cooling the condensable gas (e.g., water or the liquid fuel) incorporated into the first outflow gas can be reduced. In addition, constraint on a place where a carbon dioxide-capturing system that performs the step (I) and the step (II) is installed can be alleviated. The heating medium is not particularly limited as long as the effects of the present invention are obtained, and for example, the CO₂-enriched gas is preferably used.

In the heat exchange, the condensable gas incorporated into the first outflow gas is preferably condensed. Thus, the condensation heat of the condensable gas can be utilized in the heat exchange, and the temperature of the first outflow gas is kept constant during the condensation. Accordingly, an abrupt change in temperature thereof can be prevented. Further, the condensable gas and the remaining raw material gas incorporated into the first outflow gas can be easily separated from each other.

In one embodiment, the sweeping gas contains the CO₂-enriched gas, and the sweeping gas is captured from the second outflow gas and used as a constituent component for the raw material gas. The use of CO₂ derived from the CO₂-enriched gas (in other words, CO₂ derived from the CO₂-containing gas) as a constituent component for the raw material gas can contribute to the synthesis of a carbon neutral liquid fuel. The sweeping gas preferably contains hydrogen as a main component. Thus, the gas captured from the second outflow gas can be reused as part of the raw material gas without any need for a further separation operation.

The liquid fuel or water and the remaining raw material gas are separated and captured from the first outflow gas, and the remaining raw material gas is reused as part of the raw material gas in the step (III).

A reactor for producing the liquid fuel, the catalyst, the water vapor separation membrane, the liquid fuel separation membrane, and the sweeping gas are as described in the section A.

### Industrial Applicability

The liquid fuel production system and the method of producing a liquid fuel according to the embodiments of the present invention may each be suitably used in the production of a liquid fuel such as methanol.

### Reference Signs List

**1A, 1B** liquid fuel production system
**10** carbon dioxide-capturing portion
**20** liquid fuel-synthesizing portion
**30** temperature-controlling gas-supplying portion (sweeping gas-supplying portion)
**40** temperature-controlling gas-circulating portion (sweeping gas-circulating portion)
**50** heating medium-supplying portion
**60** heat exchanger
**70** gas A-containing gas-supplying portion (carbon dioxide-containing gas-supplying portion)
**80** first outflow gas-capturing portion
**90** raw material gas-supplying portion

## Claims

1. A liquid fuel production system, comprising:
a gas-adsorbing/desorbing portion configured to adsorb a predetermined gas A, and to desorb the gas A by being heated;
a heating medium-supplying portion configured to supply, to the gas-adsorbing/desorbing portion, a heating medium for heating the gas-adsorbing/desorbing portion;
a liquid fuel-synthesizing portion including a first gas flow path storing a catalyst that advances a conversion reaction from a raw material gas containing at least carbon dioxide and hydrogen to a liquid fuel, and a second gas flow path through which a temperature-controlling gas that controls a temperature of the first gas flow path is flowed, the liquid fuel-synthesizing portion being configured to flow a first outflow gas out of the first gas flow path, and to flow a second outflow gas out of the second gas flow path; and
a heat exchanger configured to perform heat exchange between the first outflow gas and the heating medium to heat the heating medium,
wherein the first outflow gas contains a condensable gas.

2. The liquid fuel production system according to claim 1,
wherein the gas-adsorbing/desorbing portion is a carbon dioxide-adsorbing/desorbing portion, and
wherein the liquid fuel production system further comprises a carbon dioxide-capturing portion, which includes the carbon dioxide-adsorbing/desorbing portion and is configured to capture a carbon dioxide-enriched gas from a carbon dioxide-containing gas.

3. The liquid fuel production system according to claim 2, wherein the heating medium contains the carbon dioxide-enriched gas.

4. The liquid fuel production system according to claim 1,
wherein the liquid fuel-synthesizing portion includes a water vapor separation membrane, which is configured to cause water vapor to permeate therethrough, between the first gas flow path and the second gas flow path, and
wherein the first outflow gas contains the liquid fuel.

5. The liquid fuel production system according to claim 1,
wherein the liquid fuel-synthesizing portion includes a liquid fuel separation membrane, which is configured to cause at least the liquid fuel to permeate therethrough, between the first gas flow path and the second gas flow path, and
wherein the first outflow gas contains water vapor that is a by-product of the conversion reaction.

6. The liquid fuel production system according to claim 1, wherein the temperature-controlling gas contains at least one selected from hydrogen, carbon dioxide, carbon monoxide, nitrogen, and oxygen as a main component.

7. The liquid fuel production system according to claim 2,
wherein the temperature-controlling gas contains the carbon dioxide-enriched gas, and
wherein the liquid fuel production system further comprises a temperature-controlling gas-circulating portion configured to capture the temperature-controlling gas from the liquid fuel-synthesizing portion, and to supply carbon dioxide derived from the carbon dioxide-enriched gas to the liquid fuel-synthesizing portion.

8. The liquid fuel production system according to claim 2, wherein the carbon dioxide-enriched gas is supplied as a constituent component for the raw material gas from the carbon dioxide-capturing portion to the liquid fuel-synthesizing portion.

9. A method of producing a liquid fuel, comprising:
a step (I) of bringing a carbon dioxide-containing gas into contact with a carbon dioxide adsorbent to cause the carbon dioxide adsorbent to adsorb the carbon dioxide;
a step (II) of desorbing the carbon dioxide from the carbon dioxide adsorbent through heating, followed by capture of a carbon dioxide-enriched gas;
a step (III) of performing a conversion reaction from a raw material gas containing at least carbon dioxide and hydrogen to the liquid fuel with a liquid fuel-synthesizing portion including a first gas flow path storing a catalyst that advances the conversion reaction, and a second gas flow path through which a temperature-controlling gas that controls a temperature of the first gas flow path is flowed, the liquid fuel-synthesizing portion being configured to flow a first outflow gas out of the first gas flow path, and to flow a second outflow gas out of the second gas flow path; and
a step (IV) of heating a heating medium for heating the carbon dioxide adsorbent in the step (II) through heat exchange using the first outflow gas as a heat medium,
wherein the first outflow gas contains a condensable gas.

10. The production method according to claim 9, wherein the temperature-controlling gas contains at least one selected from hydrogen, carbon dioxide, carbon monoxide, nitrogen, and oxygen as a main component.

11. The production method according to claim 9,
wherein the temperature-controlling gas contains the carbon dioxide-enriched gas, and
wherein carbon dioxide derived from the carbon dioxide-enriched gas is captured from the second outflow gas and used as a constituent component for the raw material gas.

12. The production method according to claim 9, wherein the heating medium contains the carbon dioxide-enriched gas.

13. The production method according to claim 9,
wherein the liquid fuel-synthesizing portion includes a water vapor separation membrane, which is configured to cause water vapor to permeate therethrough, between the first gas flow path and the second gas flow path, and
wherein the first outflow gas contains the liquid fuel.

14. The production method according to claim 9,
wherein the liquid fuel-synthesizing portion includes a liquid fuel separation membrane, which is configured to cause at least the liquid fuel to permeate therethrough, between the first gas flow path and the second gas flow path, and
wherein the first outflow gas contains water vapor that is a by-product of the conversion reaction.
